Europäisches Patentamt

European Patent Office

Office européen des brevets

(19)

(11) Veröffentlichungsnummer: **0 201 676**
**B1**

(12)

# EUROPÄISCHE PATENTSCHRIFT

(45) Veröffentlichungstag der Patentschrift:
07.02.90

(21) Anmeldenummer: 86102720.9

(22) Anmeldetag: 01.03.86

(51) Int. Cl.⁵: C 12 P 19/14, C 13 K 1/00

(54) Verfahren zur Herstellung eines glukosearmen Aufschlussproduktes aus inulinhaltigen Pflanzenteilen.

(30) Priorität: 08.03.85 DE 3508387

(43) Veröffentlichungstag der Anmeldung:
20.11.86 Patentblatt 86/47

(45) Bekanntmachung des Hinweises auf die Patenterteilung:
07.02.90 Patentblatt 90/06

(84) Bennante Vertragsstaaten:
BE DE FR NL

(56) Entgegenhaltungen:
DE-C-801 146
FR-A-1 284 553
FR-A-2 504 939
US-A-2 080 843
US-A-2 434 235
US-A-4 478 854

CARBOHYDRATE RESEARCH, Band 128, Nr. 2, Juni 1984, Seiten 311-320, Amsterdam, NL; A. HEYRAUD et al.: "Isolation and characterization of oligosaccharides containing d-fructose from juices of the Jerusalem artichoke. Kinetic constants for acid hydrolysis"

(73) Patentinhaber: Bärwald, Günter, Prof. Dr.-Ing.
Karmeliterweg 73/75
D-1000 Berlin 28 (DE)

(72) Erfinder: Bärwald, Günter, Prof. Dr.-Ing.
Karmeliterweg 73/75
D-1000 Berlin 28 (DE)
Erfinder: Flöther, Erhard Ferdinand
Stephanstrasse 10
D-3500 Kassel (DE)

(74) Vertreter: Reitzner, Bruno, Dr.
Patentanwälte Dipl.-Ing. R. Splanemann Dr. B. Reitzner
Tal 13
D-8000 München 2 (DE)

EP 0 201 676 B1

**Beschreibung**

Die Erfindung betrifft ein Verfahren zur Herstellung eines glukosearmen Aufschlußproduktes aus inulinhaltigen Pflanzenteilen.

Es ist bekannt, daß das in verschiedenen energieliefernden inulinhaltigen Pflanzenteilen, beispielsweise in Topinamburknollen, Dahlienknollen oder Zichorienwurzeln gespeicherte Kohlenhydrat Inulin durch Dämpfverfahren, Säurebehandlung bei Siedetemperatur oder durch Zerkleinerung und Extraktion aufgeschlossen und bis zu den Monosacchariden Fruktose und Glukose abgebaut werden kann. Die inulinhaltigen Pflanzenteile enthalten im einzelnen nicht näher beschriebene pflanzeneigene Enzyme, die Inulin bis zu Fruktose und Glukose hydrolysieren können. Reines Inulin enthält etwa 3 % Glukose und etwa 97 % Fruktose, wobei der Glukoserest sich in endständiger Position zur kettenartig in B-1,2-gebundenen polymeren Fruktose befindet. Im Knollen- und Wurzelmaterial der genannten Pflanzen liegt der Glukoseanteil höher, und zwar zwischen 5 und 15 %, weil die polymere Fruktose je nach dem Erntezeitpunkt der Knollen kurzkettiger sein können.

Bei den Säureaufschluß- und Dämpfverfahren von in Knollen oder Wurzeln gespeichertem Inulin treten erhebliche Zuckerverluste auf, und zwar besonders durch Karamelisations- und Bräunungsprodukte sowie durch Bildung von Difruktosedianhydrid.

Es ist ferner bekannt, das Inulin durch Einwirkung der in den inulinhaltigen Pflanzenteilen enthaltenen pflanzeneigenen Enzyme zu Fruktose abzubauen; die Ausbeute an Fruktose ist jedoch gering, und die Hydrolysezeit erstreckt sich über mehrere Tage. Ferner wurde überraschenderweise festgestellt, daß der Anteil der Glukose im Hydrolysat höher ist als im Ausgangsmaterial. Dies beruht wahrscheinlich darauf, daß die Fruktose durch katalytische Wirkung der pflanzeneigenen Enzyme zu Glukose isomerisiert wird. Der Vorteil, der durch den enzymatischen Abbau durch die pflanzeneigenen Enzyme gegenüber den Säureaufschluß- und Dämpfverfahren hinsichtlich eines hohen Fruktose- und niedrigen Glukosegehalts erzielt wird, hebt sich also wieder teilweise dadurch auf, daß ein gewisser Anteil der freien Fruktose durch Isomerisierung in freie Glukose umgewandelt wird.

Ein höherer Glukosegehalt ist insbesondere dann störend, wenn das Aufschlußprodukt als Diätprodukt bzw. als Diabetikerkost oder als Zwischenprodukt zur Herstellung von reiner Fruktose oder Fruktose-Oligomeren verwendet werden soll.

Es ist ferner aus der US-A-4 478 854 bekannt, das in inulinhaltigen Pflanzenteilen enthaltene Inulin zwecks Gewinnung von Ethanol durch Inulinase in einer Brennereimaische zu vergärbaren Monosacchariden zu hydrolysieren. Hierbei wird aus gewaschenen und zerkleinerten Topinamburknollen durch Vermischen mit Wasser im Verhältnis 1 : 1 eine Maische hergestellt, die zwecks Pasteurisation etwa 1 Stunde bei etwa 85°C gehalten, auf einen pH-Wert von 4,5 eingestellt und nach dem Abkühlen nach Hinzufügen der Inulinase simultan verzuckert (vollständig bis zu den Monosacchariden Fruktose und Glukose) und vergoren wird.

Ferner ist aus der FR-A-2 504 939 ein Verfahren zur Herstellung von Fruktose aus Inulin mit Hilfe eines Inulinase-Präparats mit einem Gewichtsverhältnis zwischen Endo- und Exo-Inulinase von 1 : 10 bis 10 : 1 bekannt. Bezweckt wird eine vollständige enzymatische Hydrolyse des Inulins bis zu den Monosacchariden. Für die enzymatische Hydrolyse wird ein gereinigter Extrakt verwendet. Das Hydrolyseprodukt enthält noch einen verhältnismäßig hohen Anteil an Glukose.

Der Erfindung liegt die Aufgabe zugrunde, ein Verfahren zur Herstellung eines glukosearmen Aufschlußproduktes aus einer wäßrigen Suspension von zerkleinerten inulinhaltigen Pflanzenteilen zur Verfügung zu stellen, wobei das Inulin, ohne daß es von der Suspension bzw. dem Preßsaft oder dem Extrakt abgetrennt wird, in hoher Ausbeute zu Fruktose und einem hohen Anteil an Fruktose-Oligomeren abgebaut wird, ohne daß sich in einer Neben- oder Folgereaktion freie Glukose bildet.

Diese Aufgabe wird nach dem erfindungsgemäßen Verfahren dadurch gelöst, daß man die Suspension bzw. einen daraus gewonnenen Preßsaft oder Extrakt bei Temperaturen bis zu 100°C erhitzt, bis die pflanzeneigenen Enzyme weitgehend inaktiviert sind, worauf man der Suspension bzw. dem Preßsaft oder dem Extrakt nach der Abkühlung Inulinase zusetzt, wobei man den Aufschluß so lenkt, daß auf ein Gewichtsteil Fruktose-Oligomer (mit 2 bis 10 Fruktoseeinheiten) 1,5 bis 3 Gewichtsteile Fruktose und 0 bis 1,5 Gewichtsteile Inulin entfallen, und daß auf 1 Gewichtsteil Fruktose bis 0,21 Gewichtsteile Glukose entfallen.

Obwohl das erfindungsgemäße Verfahren auf alle inulinhaltigen Pflanzenteile anwendbar ist, verwendet man als inulinhaltige Pflanzenteile vorzugsweise Topinamburknollen, Dahlienknollen oder Zichorienwurzeln, die in größeren Mengen als Handelsprodukte zur Verfügung stehen.

Die erfindungsgemäß verwendeten Inulinasen sind an sich bekannt (vgl. beispielsweise "Starch/Stärke", 1981, S. 373 - 377; J. Chem. Tech. Biotechnol. 1984, 34B, 45 - 51). Auch ihre Anwendung beim Abbau von Inulin aus inulinhaltigen Pflanzenteilen zu Fruktose ist bekannt. Nicht bekannt ist jedoch ihre Anwendung zur Herstellung von glukosearmen Aufschlußprodukten und Fruktose-Oligomeren.

Die Fruktose-Oligomeren stellen lösliche Ballaststoffe dar, die wie das Inulin die geringste insulinogene Wirkung zeigen. Im Gegensatz zu reiner Fruktose ergibt ein Gemisch aus Fruktose und Fruktose-Oligomeren einen geringeren Triglyceridspiegel im Blut. Daher wird das Gewichtsverhältnis Fruktose : Fruktose-Oligomere zu Inulin auf 1,5 - 3 : 1 : 0 bis 1,5 eingestellt. Unter Fruktose-Oligomeren sind hierbei solche mit 2 bis 10 Fruktoseeinheiten zu verstehen.

Vorzugsweise verwendet man erfindungsgemäß aus Schimmelpilzen der Gattungen Aspergillus, Penicillium und Fusarium und/oder aus bestimmten Hefen der Gattungen Kluyveromyces, Saccharomyces und Candida gewonnene Inulinasen, wobei die Auswahl jedoch nicht auf diese Arten beschränkt ist.

Die Inulinasen wirken in einem breiten pH- und Temperaturbereich, wobei die Reaktionsgeschwindigkeit von der Substratkonzentration, der Enzymmenge sowie den für die jeweilige Inulinase spezifischen pH- und Temperaturoptima abhängt. Die optimalen pH-Bedingungen beim Aufschluß von Topinamburknollen liegen bei etwa 5,1. Es ist ferner bekannt, daß die Inulinasen exogene und endogene Hydrolysefunktionen haben, wobei die endogenen Inulinasen das langkettige Inulinmolekül zu kürzerkettigen Untereinheiten, die aus Fruktose-Oligomeren bestehen, hydrolysiert, während die exogenen Inulinasen zunächst die endständigen Glukoseeinheiten und anschließend die jeweils folgenden endständigen Fruktoseeinheiten vom Inulin abspalten. Erfindungsgemäß werden die endogenen Inulinasen bevorzugt.

Bei der Durchführung des erfindungsgemäßen Verfahrens erfolgt zunächst eine mechanische Zerkleinerung der inulinhaltigen Pflanzenteile, wobei die Zellen teilweise zerstört werden, damit für den sich anschließenden enzymatischen Aufschluß das Inulin als Substrat zur Verfügung steht. Die Zerkleinerung der Pflanzenteile kann beispielsweise mit Hilfe von Sieb- oder Hammermühlen oder mit Hilfe von Schnitzelwerken durchgeführt werden, wobei die Zerkleinerung auf eine Teilchengröße zwischen 0,5 und 25 mm erfolgt. Beispielsweise wurden Hammermühlen mit Siebweiten von 1,0 und 1,5 mm erprobt, desgleichen auch Schnitzelwerke für Zuckerrüben mit Schnitzeldurchmessern von 25 mm. Die zerkleinerten Pflanzenteile können nun entweder extrahiert oder weiter zu einem Mus zerkleinert werden, welches durch gegebenenfalls Zusatz von Wasser in eine fließfähige Suspension übergeführt wird. Der Anteil des Wassers beträgt üblicherweise 10 bis 180 %, vorzugsweise 20 bis 50 %, der inulinhaltigen Pflanzenteile.

Zur Inaktivierung der pflanzeneigenen Enzyme wird der pH-Wert der Suspension bzw. des wäßrigen Extraktes im allgemeinen auf 3,0 bis 7,0, vorzugsweise auf 3,5 bis 5,5 eingestellt, worauf die Suspension bzw. der wäßrige Extrakt im allgemeinen auf 70 bis 100°C, vorzugsweise auf 80 bis 90°C, erhitzt wird. Die Erhitzungsdauer liegt je nach der angewendeten Temperatur zwischen etwa 10 Minuten und 1 Stunde, vorzugsweise bei etwa 30 Minuten. Eine Erhitzung auf über 100°C ist zu vermeiden, da bei diesen hohen Temperaturen, wie schon gesagt, erhebliche Zuckerverluste durch Karamelisations- und Bräunungsreaktionen sowie durch Bildung von Difruktosedianhydrid auftreten.

Nach dem Erhitzen wird die Suspension bzw. der Preßsaft oder der Extrakt auf Temperaturen von etwa 30 bis 80°C, vorzugsweise auf Temperaturen zwischen 40 und 65°C, abgekühlt und üblicherweise mit Säuren (bei Lebensmittelendprodukten mit den zugelassenen Genußsäuren) auf einen pH-Wert von etwa 3 bis 6 eingestellt. Dann werden die Inulinasen zugesetzt. Die Einwirkungsdauer hängt von der Temperatur, der verwendeten Inulinase und dem gewünschten Aufschlußgrad ab. Wünscht man beispielsweise Fruktose-Oligomere, so verwendet man bevorzugt endogene Inulinase bzw. hält die Einwirkungszeit kürzer als bei der Erzeugung von monomerer Fruktose.

Für den enzymatischen Aufschluß werden vorzugsweise die im optimalen Wirkbereich liegenden pH- und Temperaturverhältnisse angewendet. Zum Beispiel liegen die pH-Werte bei Verwendung von Inulinasen aus Candida- oder Kluyveromyces-Hefen zwischen 3,5 und 5,5 und die Temperaturen zwischen 35 und 55°C; bei Verwendung von Inulinasen aus Schimmelpilzen liegen die pH-Werte zwischen 3,5 und 7,0 und die Temperaturen zwischen 40 und 65°C.

Der enzymatische Aufschluß kann durch Erhitzen der Suspension bzw. des Preßsaftes oder des Extraktes unterbrochen werden, wobei die Inulinase desaktiviert wird. Das Erhitzen kann mit dem Eindampfen (beispielsweise bei 80°C im Vakuum) kombiniert werden, wenn man beispielsweise ein konzentriertes Aufschlußprodukt erhalten will.

Nach einer bevorzugten Ausführungsform der Erfindung kann der Glukosegehalt des Aufschlußproduktes dadurch vermindert werden, daß man die endständigen Glukosegruppen des Inulins durch Begasen der Suspension bzw. des Preßsaftes oder des Extraktes mit Sauerstoff oder einem sauerstoffhaltigen Gas unter Zusatz von Glukoseoxidase und Katalase zu Glukoronsäure oxidiert. Ein derartiges Enzymgemisch ist aus der DE-PS-2 520 792 bekannt. Dort wird es als Oxidationsschutzmittel zur Entfernung von Sauerstoff aus vergorenen und nichtvergorenen Getränken, denen gegebenenfalls Glukose zugesetzt ist, verwendet. Im vorliegenden Fall dient es dagegen zur katalytischen Oxidation der Glukose. Das Enzymgemisch kann an einem inerten Adsorptionsmittel, wie Silikagel, mit Siliciumhydroxid beschichtetem porösem Glas, Aluminiumoxid, Silikaten (insbesondere Bentonit) oder Zeolithen, adsorbiert sein. Das Enzymgemisch auf Glukoseoxidase und Katalase sowie der Sauerstoff bzw. das sauerstoffhaltige Gas können dem wäßrigen Medium vor, während oder nach dem enzymatischen Aufschluß mit Inulinase zugesetzt werden. Die Konzentration des im wäßrigen Medium gelösten Sauerstoffs wird vorzugsweise zwischen 0,1 und 1 mg $O_2$/Liter eingestellt. Es handelt sich hierbei um verhältnismäßig niedrige Sauerstoffkonzentrationen, so daß eine Oxidationsschädigung der anderen Inhaltsstoffe (z. B. Vitamine und Aromastoffe) des Aufschlußproduktes vermieden wird.

Gegenstand der Erfindung ist ferner die Verwendung der nach dem erfindungsgemäßen Verfahren erhaltenen Aufschlußprodukte als Diätprodukte, als Diabetikerkost und/oder als Zwischenprodukte zur Gewinnung von Fruktose oder Fruktose-Oligomeren. Vorzugsweise werden die erfindungsgemäßen Aufschlußprodukte in Form von Preßsäften oder Preßsaft-Konzentraten verwendet.

Das erfindungsgemäße Verfahren ist so schonend, daß empfindliche Substanzen, die in den inulinhaltigen Pflanzenteilen enthalten sind, nicht geschädigt werden. Hierzu zählen beispielsweise Vitamine, Proteine, ungesättigte Fette und Aromastoffe.

Die Erfindung ist durch die nachstehenden Beispiele erläutert.

**Beispiel 1**

Es wurden 3,6 Tonnen Topinamburknollen (zuvor gründlich gewaschen) auf einer Fräse so mechanisch zerkleinert, daß die Partikelgröße vorzugsweise unter 1,5 mm lag. Dem Mus wurden 1 400 Liter Wasser zugefügt, damit es sich pumpen und rühren läßt. Die Suspension hatte einen anfänglichen pH-Wert von 6,3; es wurde über den Prozeß hinweg mit fester Zitronensäure (Monohydrat) soweit angesäuert, daß der pH-Wert zwischen 4,5 und 5 lag. Durch Rohrbündel-Wärmetauscher wurde die Suspension direkt nach dem mechanischen Aufschluß auf 80°C erhitzt, 30 Minuten auf dieser Temperatur gehalten und im Gegenstromprinzip auf 60°C gekühlt. Die Suspension wurde zur Hydrolyse mit 3 Einheiten Inulinase pro Gramm Inulin (der Inulingehalt der Knollen war zuvor ermittelt worden) versetzt. Die in der Suspension gebildeten Mengen an freien Monosacchariden Fruktose und Glukose wurden mittels Chromatographie (HPLC) sowie biochemisch (enzymatische Methoden, Tests nach Boehringer) bestimmt. In einem zweiten Versuch wurden 1 250 kg Topinamburknollen mechanisch wie zuvor zerkleinert und mit 485 Liter Wasser suspendiert. Diese Suspension wurde jedoch nur auf 60°C erwärmt unter Einstellung des pH-Wertes auf 4,5 bis 5. Die Ensymdosierung erfolgte wie zuvor. Die Bildung von Monosacchariden Fruktose und Glukose wurde, wie zuvor beschrieben, quantitativ festgestellt. Die Versuchsergebnisse sind in Tabelle I angegeben.

**Tabelle I**

| | | Fruktose g pro kg Knollen | Glukose g pro kg Knollen | Verhälnis Fruktose:Glukose | freie Monosaccharide in Prozent Gesamtzucker der Knollen (Verzuckerungsgrad) |
|---|---|---|---|---|---|
| A. | nach mechanischem Aufschluß | 1,2 | 0,4 | 1 : 0,33 | 0,9 |
| | nach dem Erhitzen auf 80°C (Inaktivierung der pflanzeneigenen Enzyme) 1 Std. enzymatische Hydrolyse | 34,2 | 7,2 | 1 : 0,21 | 23,7 |
| | wie zuvor, doch nach 2 Std. enzymatischer Hydrolyse | 48,7 | 9,1 | 1 : 0,18 | 33,0 |
| B. | ohne Erhitzung (keine Inaktivierung der pflanzeneigenen Enzyme) 1 Std. enzymatische Hydrolyse bei 60°C | 31,5 | 8,2 | 1 : 0,26 | 22,7 |
| | wie zuvor, doch nach 2 Std. enzymat. Hydrolyse | 42,2 | 11,9 | 1 : 0,28 | 30,9 |

Die Versuchsergebnisse zeigen, daß das Verhältnis Fruktose : Glukose am höchsten ist, wenn die pflanzeneigenen Enzyme vor der Behandlung mit Inulinase inaktiviert wurden.

**Beispiel 2**

Aus einer nach Beispiel 1 (Versuch A; 2 Std. enzymatische Hydrolyse) behandelten Topinambur-Suspension wurde ein Preßsaft (spez. Gewicht = 1 070; pH-Wert = 4,7) hergestellt. Der Preßsaft wurde im Fallstromverdampfer bei Temperaturen unter 65°C unter Vakuum auf das spezifische Gewicht 1 070 eingeengt. 250 ml des Preßsaftes wurden bei 50°C mit handelsüblicher Glukoseoxidase/Katalase (GOD 155) versetzt, welche in der Flüssigkeit suspendiert wurde. Mittels einer Fritte wurde ständig Luft von unten in das Gefäß eingeleitet, wobei der Luftstrom so bemessen war, daß maximal 1 mg $O_2$/Liter (mit einem Oxi-Sauerstoffmeßgerät und eine Sauerstoffelektrode gemessen) gelöst waren. Die Abnahme des Glukosegehalts ist in Tabelle II angegeben.

**Tabelle II**

Beginn:      13 g Glukose/Liter      Topinambur-Preßsaft
nach 1 Std.    5 g "                                         "
nach 2 Std.  2,5 g "                                       "
nach 3 Std.    1 g "                                        "

Der erhaltene Preßsaft ist also verhältnismäßig arm an Glukose und kann als Getränk für Diabetiker verwendet werden.

Vergleichsbeispiel

Es wurden Topinamburknollen über ein Sieb mit 1,5 mm Schlitzbreite gerieben. Dem Knollenbrei wurde gewichtmäßig die Hälfte an Wasser zugesetzt. Ein Teil des so verdünnten Breis wurde unter Rühren bei 18°C und ein anderer Teil bei 41°C zur Hydrolyse stehengelassen. Die gebildeten Mengen an Fruktose und Glukose wurden bestimmt und auf die Trockenmasse des Knollenmaterials umgerechnet. Die Hydrolysetemperatur von 41°C entspricht der optimalen Inulinase. Die Ergebnisse sind in Tabelle III engegeben.

Tabelle III

| Einwirkzeit | Nullprobe | 2 Tage | 5 Tage | 9 Tage |
|---|---|---|---|---|
| Fruktosegehalt, g pro kg Knollen- trockenmasse | 41°C : 1,7 | 145 | 246 | 366 |
| | 18°C : 1,7 | 31 | 66 | nicht bestimmt |
| Glukosegehalt, g pro kg Knollen- trockenmesse | 41°C : 0,2 | 41 | 86 | 131 |
| | 18°C : 0,2 | 9 | 23 | nicht bestimmt |

Die auf Trockenmasse bezogenen Angaben wurden durch Verdunstungsverluste notwendig, die sich aufgrund der langen Stehzeit bei den angegebenen Temperaturen ergaben.

Man erkennt eine verstärke Glukosebildung, die wahrscheinlich dadurch bedingt ist, daß in dem wäßrigen Material aus zerkleinerten Knollen Isomerisierungsreaktionen ablaufen, die durch die pflenzeneigenen Enzyme katalysiert werden.

Umittelbar nach dem mechanischen Aufschluß der Topinamburknollen wird ein Verhältnis von 1,7 Masseteilen Fruktose zu 0,2 Masseteilen Glukose analytisch festgestellt; dies entspricht einem Verhältnis von etwa 88,2 % Fruktose zu etwa 11,8 % Glukose oder einem Fruktose/Glukose-Quotienten von 1 : 0,12. Im Verlauf der Hydrolyse steigt der Anteil an freier Fruktose an. Aufgrund der Struktur des Inulins (endständige Glukosegruppen) müßte sich, wenn ausschließlich eine Hydrolyse stattfinden würde, das Verhältnis zwischen den Monosacchariden zugunsten der Fruktose ändern. Es tritt jedoch das Gegenteil ein, d. h. es wird unverhältnismäßig viel freie Glukose gebildet. Nach 2 Tagen beträgt der Fruktose/Glukose-Quotient (bei 41°C) 1 : 0,28, nach 5 Tagen sogar 1 : 0,35. Dieser Befund kann nur durch eine Isomerisierung der Fruktose zu Glukose unter der Einwirkung der pflanzeneigenen Enzyme erklärt werden.

Patentansprüche

1. Verfahren zur Herstellung eines glukosearmen Aufschlußproduktes aus einer wäßrigen Suspension von zerkleinerten inulinhaltigen Pflanzenteilen, wobei das Inulin, ohne daß es von der Suspension bzw. dem Preßsaft oder dem Extract abgetrennt wird, zu Fruktose und Fruktose-Oligomeren abgebaut wird, dadurch gekennzeichnet, daß man die Suspension bzw. einen daraus gewonnenen Preßsaft oder Extrakt bei Temperaturen bis zu 100°C erhitzt, bis die pflanzeneigenen Enzyme weitgehend inaktiviert sind, worauf man der Suspension bzw. dem Preßsaft oder dem Extrakt nach der Abkühlung Inulinase zusetzt, wobei man den Aufschluß so lenkt, daß auf ein Gewichtsteil Fruktose-Oligomer (mit 2 bis 10 Fruktoseeinheiten) 1,5 bis 3 Gewichtsteile Fruktose und 0 bis 1,5 Gewichtsteile Inulin entfallen und daß auf ein Gewichtsteil Fruktose bis 0,21 Gewichtsteile Glukose entfallen.

2. Verfahren nach Anspruch 1, dadurch gekennzeichnet, daß man als inulinhaltige Pflanzenteile Topinamburknollen, Dahlienknollen oder Zichorienwurzeln verwendet.

3. Verfahren nach Anspruch 1 oder 2, dadurch gekennzeichnet, daß man aus Schimmelpilzen der Gattungen Aspergillus, Penicillium und Fusarium und/oder aus Hefen der Gattungen Kluyveromyces, Saccharomyces und Candida gewonnene Inulinase verwendet.

4. Verfahren nach einem der Ansprüche 1 bis 3, dadurch gekennzeichnet, daß man die Zerkleinerung der Pflanzenteile mit Hilfe von Sieb- oder Hammermühlen oder mit Hilfe von Schnitzelwerken bis auf eine Teilchengröße zwischen 0,5 und 25 mm durchführt.

5. Verfahren nach einem der Ansprüche 1 bis 4, dadurch gekennzeichnet, daß man den pH-Wert der Suspension auf 3,0 bis 7,0, vorzugsweise auf 3,5 bis 5,5, einstellt und die Suspension bzw. den Preßsaft oder Extrakt auf 70 bis 100°C, vorzugsweise auf 80 bis 90°C, erhitzt.

6. Verfahren nach einem der Ansprüche 1 bis 5, dadurch gekennzeichnet, daß man die Inulinase bei Temperaturen zwischen 40 und 65°C zusetzt.

7. Verfahren nach einem der Ansprüche 1 bis 6, dadurch gekennzeichnet, daß man die endständigen Glukosegruppen aus dem Inulin durch Begasen der Suspension oder des Preßsaftes bzw. des Extraktes mit Sauerstoff oder einem sauerstoffhaltigen Gas unter Zusatz von Glukoseoxidase und Katalase zu Glukonsäure oxidiert.

8. Verfahren nach Anspruch 7, dadurch gekennzeichnet, daß man die Konzentration des im wäßrigen Medium gelösten Sauerstoffs zwischen 0,1 und 1 mg $O_2$/Liter einstellt.

9. Verwendung des nach einem der Ansprüche 1 bis 8 erhaltenen Aufschlußproduktes als Diätprodukt, als Diabetikerkost und/oder als Zwischenprodukt zur Gewinnung von Fruktose oder Fruktose-Oligomeren.

10. Verwendung nach Anspruch 9 in Form eines Preßsaftes oder Preßsaft-Konzentrates.

## Claims

1. A process for the preparation of a low-glucose digestion product from an aqueous suspension of comminuted inulin-containing parts of plants, the inulin being degraded to fructose and fructose oligomers, without being separated from the suspension or the pressed juice or the extract, characterized in that the suspension or a pressed juice or extract obtained therefrom are heated at temperatures of up to 100°C until the enzymes intrinsic to the plant have substantially being inactivated, whereupon, after cooling, inulinase is added to the suspension or the pressed juice or the extract, the digestion being controlled so that 1.5 to 3 parts by weight of fructose and 0 to 1.5 % by weight are present for one part by weight of fructose oligomer (with 2 to 10 fructose units), and that up to 0.21 parts by weight of glucose are present for one part by weight of fructose.

2. Process according to claim 1, characterized in that Jerusalem artichoke tubers, dahlia tubers or chicory roots are used as inulin-containing parts of plants.

3. Process according to claims 1 or 2, characterized in that inulinase obtained from molds of the genera Aspergillus, Penicillium and Fusarium and/or from yeasts of the genera Kluyveromyces, Saccharomyces and Candida are used.

4. Process according to anyone of claims 1 to 3, characterized in that the comminution of the parts of plants is carried out with the use of screening or hammer mills or with the use of slicing machines down to a particle size between 0.5 and 25 mm.

5. Process according to anyone of claims 1 to 4, characterized in that the pH of the suspension is adjusted to 3.7 to 7.0, preferably to 3.5 to 5.5, and the suspension or the pressed juice or extract is heated at 70 to 100°C, preferably at 80 - 90°C.

6. Process according to anyone of claims 1 to 5, characterized in that the inulinase is added at temperatures between 40 and 65°C.

7. Process according to anyone of claims 1 - 6, characterized in that the terminal glucose groups in the inulin are oxidized to gluconic acid by passing oxygen or an oxygen-containing gas through the suspension or the pressed juice or extract, with the addition of glucose oxidase and catalase.

8. Process according to claim 7, characterized in that the concentration of the oxygen dissolved in the aqueous medium is adjusted to between 0.1 and 1 mg $O_2$/liter.

9. The use of the digestion product obtained according to anyone of claims 1 to 8 as dietetic products, as food for diabetics and/or as intermediates for obtaining fructose or fructose oligomers.

10. The use according to claim 9 in the form of a pressed juice or pressed juice concentrate.

## Revendications

1. Procédé de fabrication d'un produit de digestion à faible teneur en glucose à partir d'une suspension aqueuse d'éléments végétaux fragmentés à teneur en inuline, suivant lequel l'inuline, sans être séparée de la suspension ou bien du jus de pression ou de l'extrait, est décomposée en fructose et en oligomères du fructose, caractérisé par le fait qu'on chauffe la suspension ou bien un jus de pression ou un extrait obtenu à partir de celle-ci, à des températures allant jusqu'à 100°C, jusqu'à ce que les enzymes propres aux végétaux soient inactivés dans une large mesure, sur quoi on ajoute de l'inulinase à la suspension ou bien au jus de pression ou à l'extrait après le refroidissement, la digestion étant conduite de telle sorte qu'à une partie en poids d'oligomère du fructose (comportant 2 à 10 unités fructose), il revienne 1,5 à 3 parties en poids de fructose et 0 à 1,5 partie en poids d'inuline, et qu'à une partie en poids de fructose, il revienne jusqu'à 0,21 partie en poids de glucose.

2. Procédé selon la revendication 1, caractérisé par le fait qu'on utilise, comme éléments végétaux à teneur en inuline, des tubercules de topinambour, des tubercules de dahlia ou des racines de chicorée.

3. Procédé selon l'une des revendications 1 et 2, caractérisé par le fait qu'on utilise de l'inulase obtenue à partir de moisissures des genres **Aspergillus, Penicillium** et **Fusarium** et/ou à partir de levures des genres **Kluyveromyces, Saccharomyces** et **Candida**.

4. Procédé selon l'une des revendications 1 à 3, caractérisé par le fait qu'on effectue la fragmentation des éléments végétaux à l'aide de broyeurs-cribleurs ou de broyeurs à marteaux, ou à l'aide de coupe-racines jusqu'à une grosseur de particule se situant entre 0,5 et 25 mm.

5. Procédé selon l'une des revendications 1 à 4, caractérisé par la fait qu'on règle le pH de la suspension à une valeur de 3,0 à 7,0, de préférence, à une valeur de 3,5 à 5,5, et qu'on chauffe la suspension ou bien le jus de pression ou extrait à une température de 70 a 100°C, de préférence, de 80 à 90°C,

6. Procédé selon l'un des revendications à 5, caractérisé par la fait qu'on ajoute l'inulase à des températures comprises entre 40 et 65°C,

7. Procédé selon l'une des revendications 1 à 6, caractérisé par le fait qu'on oxyde en acide gluconique les groupes glucose terminaux provenant de l'inuline en faisant absorber de l'oxygène ou un gaz renfermant de l'oxygène à la suspension ou au jus de pression ou bien à l'extrait, sous addition de glucose-oxydase et de catalase.

8. Procédé selon la revendication 7, caractérisé par le fait qu'on règle la concentration de l'oxygène dissous dans le milieu aqueux à une valeur comprise entre 0,1 et 1 mg d'$O_2$/litre.

9. Utilisation du produit de digestion obtenu conformément à l'une des revendications 1 à 8, comme produit diététique, aliment pour diabétiques et/ou comme produit intermédiaire pour l'obtention de fructose ou d'oligomères du fructose,

10. Utilisation selon la revendication 9 sous la forme d'un jus de pression ou d'un concentré de jus de pression.